(19) Europäisches Patentamt
European Patent Office
Office européen des brevets

(11) Publication number: 0 477 853 A1

# (12) EUROPEAN PATENT APPLICATION

(21) Application number: 91116229.5

(22) Date of filing: 24.09.91

(51) Int. Cl.5: C07C 205/38, C07C 201/12, C07C 201/08, C07C 217/90, C07C 213/02

(30) Priority: 28.09.90 IL 95848

(43) Date of publication of application: 01.04.92 Bulletin 92/14

(84) Designated Contracting States: DE ES FR GB NL

(71) Applicant: Bromine Compounds Ltd.
Makleff House P.O.B. 180
Beer-Sheva 84101(IL)

(72) Inventor: Zviely, Michael
3 Haim-Hazaz street
Savioney-Dania, Haifa 34984(IL)
Inventor: Ioffe, David
46/19 Gutel Levin
Ramot Sapir, Haifa 33922(IL)
Inventor: Zilberman, Joseph
20/10 Hailan Street
Acco 24411(IL)

(74) Representative: Perani, Aurelio et al
c/o JACOBACCI-CASETTA & PERANI S.p.A 7,
Via Visconti di Modrone
I-20122 Milano(IT)

## (54) Process and novel intermediate for the preparation of 3,4'-diaminodiphenyl ether.

(57) In a process for the preparation of 3,4'-diaminodiphenyl ether, 3-nitrodiphenyl ether is reacted with a compound selected from $Br_2$ and $HNO_3$ to yield a compound of the formula:

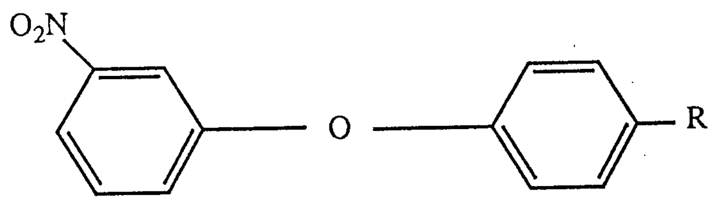

wherein

R is Br or $NO_2$; and then carrying out, in any appropriate order, the steps of: (a) substituting any Br atom in the 4'-position with an $NH_2$ group; (b) reducing any $NO_2$ group to an $NH_2$ group.

EP 0 477 853 A1

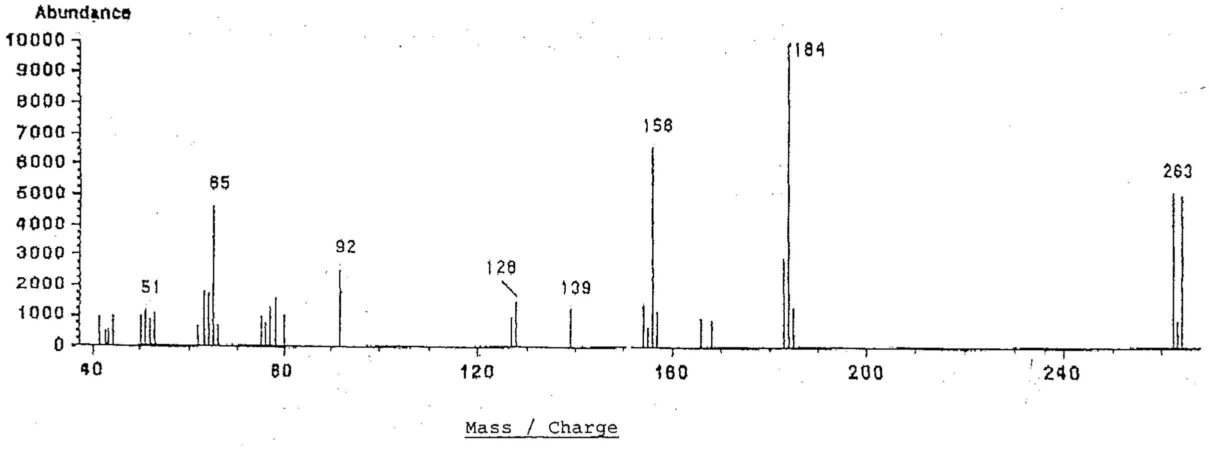
Abundance
10000
9000
8000
7000
6000
5000
4000
3000
2000
1000
0
184
158
263
65
92
128
139
51
40
80
120
160
200
240
Mass / Charge

The present invention relates to a process and a novel intermediate for the preparation of 3,4'-diaminodiphenyl ether. More particularly, the invention relates to a method by means of which 3,4'-diaminodiphenyl ether may be prepared with high purity and in high yield.

3,4'-Diaminodiphenyl ether (3,4'-DADPE) is an important monomer useful for the manufacture of high performance polymers. Many processes are known in the art for preparing this compound, some of which, however, suffer from various drawbacks. Processes of the art are complicated, expensive and involve difficultly obtainable starting materials. Furthermore, because of the use made of this compound very stringent requirements are imposed on its purity, which are not always met by the known processes.

Some such processes will be described hereinafter. Japan Kokai JP 58 157749 describes a synthesis of 3,4'-diaminodiphenyl ether by the Ullmann reaction of 3,4-dichloronitrobenzene and 2,4-dichlorophenol, to obtain the corresponding trichloro derivative, which is further nitrated with $HNO_3$ at the 3-position. This trichloro-dinitro derivative is further hydrogenated to obtain 3,4'-DADPE.

Another method of preparation, described in JP 01 85951, involves the treatment of 3,4'-dichlorodiphenyl ether with ammonia at 184 °C and 33 atmospheres. In Japanese Patent JP 60 105,649, 3,4'-DADPE is prepared in a 2-stage process, in which the first step is the "Anti-Ullmann" reaction of 3-aminophenol and 4-chloronitrobenzene (etherification without a copper catalyst), and the second step involves the hydrogenation of 3-amino-4'-nitrodiphenyl ether. In a similar process 3,4'-DADPE is prepared from 3-nitrophenol instead of 3-aminophenol as starting material (JP 61 225,155). 3-Amino-4'-nitrodiphenyl ether was hydrogenated on 5% Pd-C to yield 3,4'-DADPE, in Japanese Patent JP 60 149,549.

It has now been found, and this is an object of the present invention, that it is possible to provide a simple and relatively inexpensive process for the preparation of 3,4'-diaminodiphenyl ether, starting from readily available materials.

It has further been found, and this is another object of the invention, that it is possible to obtain 3,4'-diaminodiphenyl ether not only with high yield but in very high purity.

The process for the preparation of 3,4'-diaminodiphenyl ether according to the invention comprises reacting 3-nitrodiphenyl ether with a compound selected from $Br_2$ and $HNO_3$ to yield a compound of the formula:

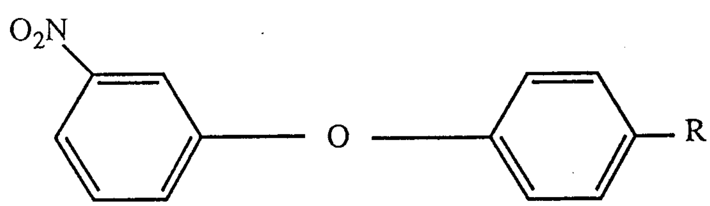

wherein

R is Br or $NO_2$; and then carrying out, in any appropriate order, the steps of:

(a) substituting any Br atom in the 4'- position with an $NH_2$ group;

(b) reducing any $NO_2$ group to an $NH_2$ group.

3-Nitrodiphenyl ether (NDPE) is readily available as it can be prepared in a simple manner, e.g., according to F. Ullmann et al., Justus Liebig Annalen Der Chemie, 350, 83, 1906.

The invention is based on a process which, generally speaking, involves functionalizing the 4'-position of the starting material 3-nitrodiphenyl ether, replacing the moiety at the 4'- position with an amino group, in the case of 4'-bromo substituent, and reducing the nitro group to an amino group, in the case of 4'-nitro substituent. Thus, the process that will be described hereinafter is made possible by the ability to functionalize the 4'-position of this starting material in a highly selective manner.

As will be apparent to the skilled chemist, the process of the invention can be carried out according to different embodiments. Three such embodiments are shown in Schemes I, II and III below.

## Scheme I

In Scheme I, NDPE is brominated to yield the 4'-bromo derivative of formula II. This compound is aminated with $NH_3$ to yield the 3-nitro-4'-amino compound of formula III, which in turn is reduced to yield the final product.

The two last steps of the process of Scheme I can also be carried out sequentially in the same vessel, by aminating the compound of formula II and then reducing in situ the resulting product.

## Scheme II

In Scheme II, the same steps as in Scheme I are carried out, with the exception that the last two steps are inverted. Thus, the compound of formula II is first reduced to yield its amino analogue of formula IIIa, which is then aminated to substitute the bromine atom at the 4'-position.

## Scheme III

In Scheme III, 3-nitrodiphenyl ether is first reacted with $HNO_3$ to yield the dinitro derivative of formula IIIb, which is then reduced to yield the final compound.

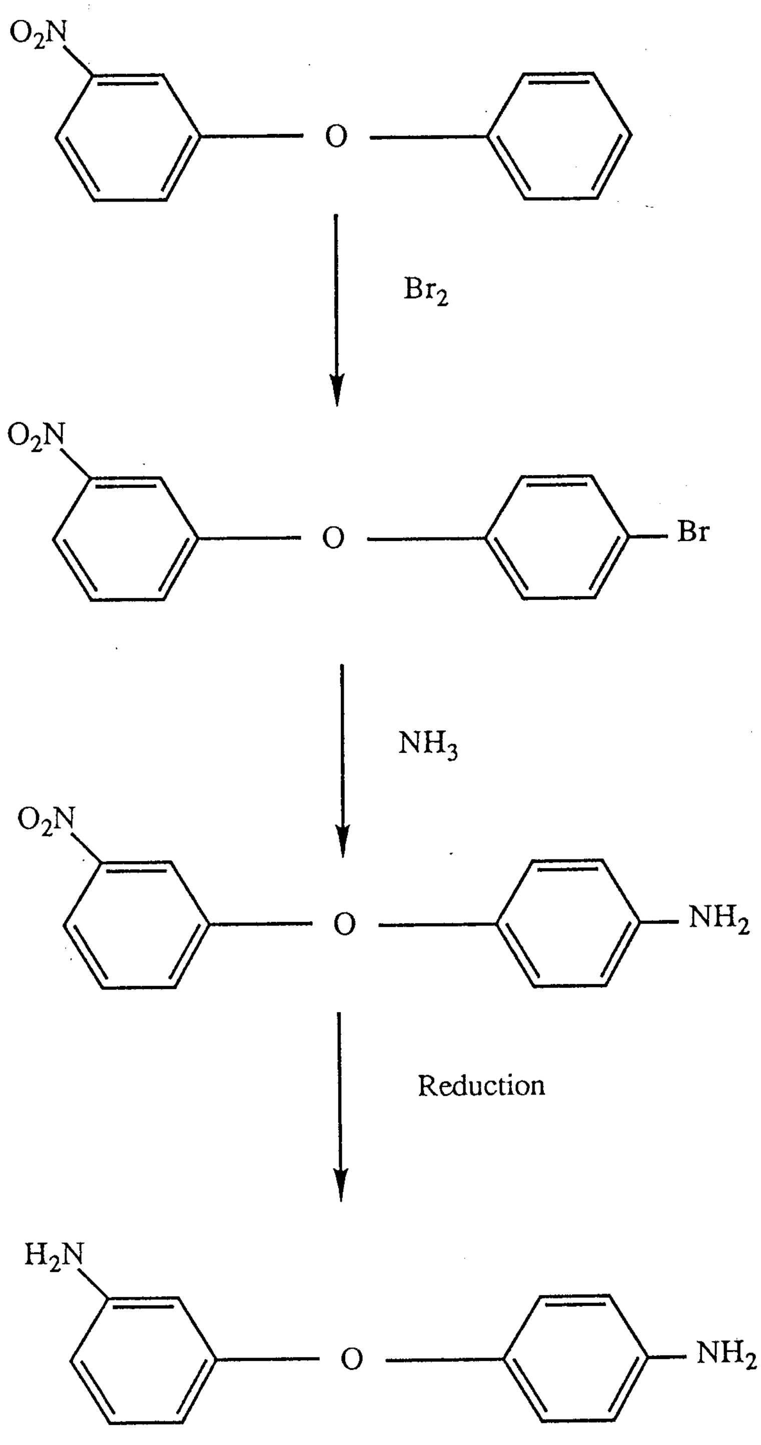


(I)

(II)

(III)

(IV)

Scheme I

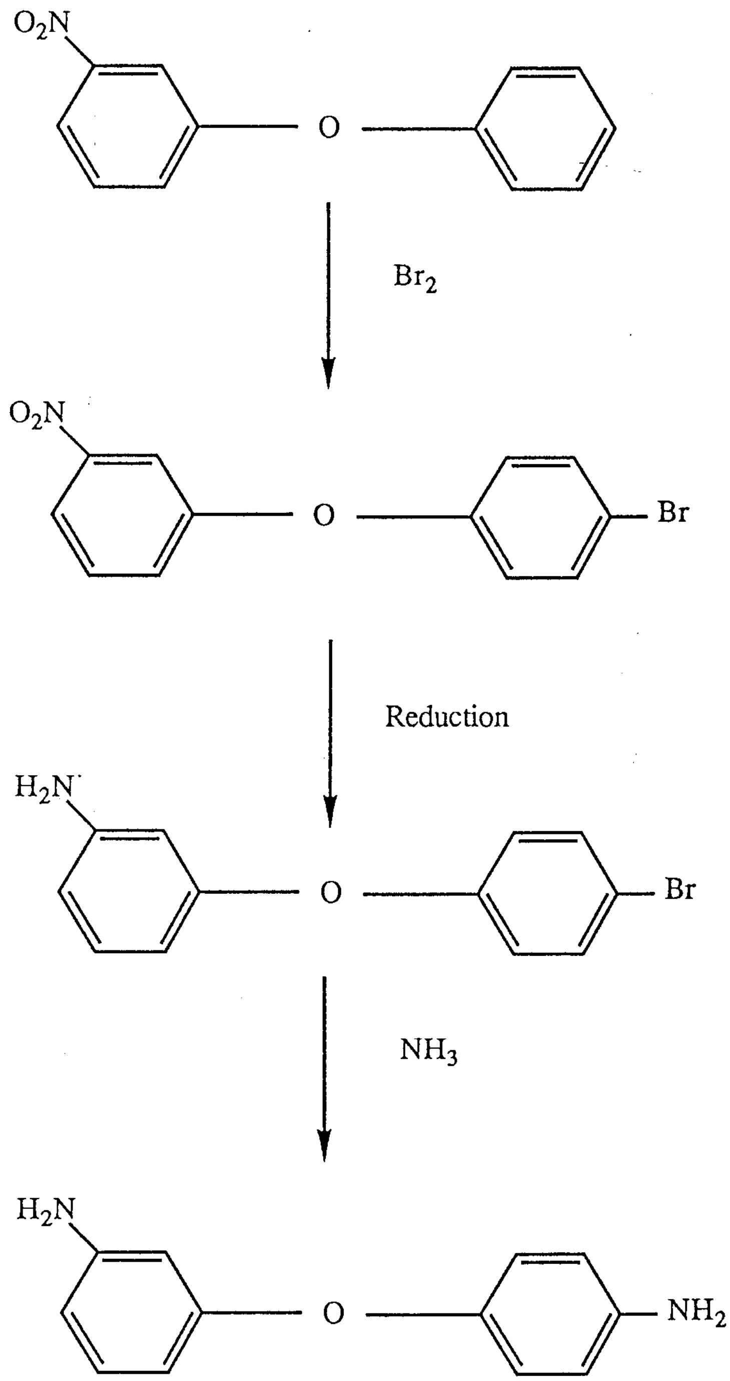


(I)

(II)

(IIIa)

(IV)

Scheme II

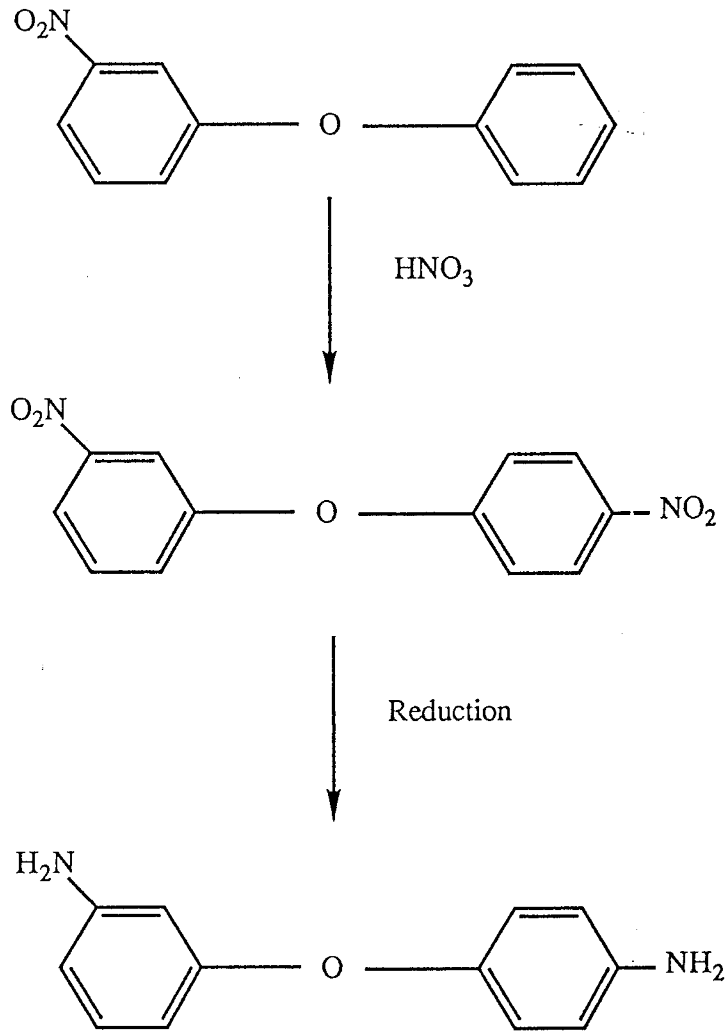


(I)

(IIIb)

(IV)

Scheme III

The compound of formula IIIa of Scheme II is a novel compound, and as such, also forms part of the present invention. As noted, this novel compound is a valuable intermediate to 3,4'-diaminodiphenyl ether.

In the processes shown in Schemes I and II the bromination reaction can be carried out without a solvent, under neat conditions, or in a solvent. A preferred solvent is a water-immiscible solvent such as dichloromethane. Many water-immiscible solvents can conveniently be used, as long as they are substantially unreactive under the reaction conditions. Preferred compounds of this type are the halogenated aliphatic hydrocarbons, and those aromatic compounds that are not substantially susceptible to bromination. When bromination reagents like N,N'-dibromo-5,5-dimethylhydantoin (DBDMH) or N-bromosuccinimide (NBS) are used, a water-immiscible solvent, e.g. acetonitrile, can be used. The bromination reaction can be carried out in a broad range of temperatures, between $^{-}10$ ° C and $^{+}70$ ° C, and is preferably carried out in a temperature range between 20 ° and 65 ° C.

Any suitable brominating agent can be employed, such as elemental bromine, hydrobromic acid, alone or in conjunction with $H_2O_2$, $NaBrO_3$ or the like oxidizing agents, or organic brominating agents, such as DBDMH and NBS.

The substitution of the bromine atom with an amino group is preferably carried out by reacting the bromine-containing intermediate with ammonia, in an aqueous solvent medium and in the presence of a copper compound catalyst. Preferably, this reaction is carried out at elevated temperatures and under

autogenous pressures, such as are normally obtained in an autoclave. Ammonia may be supplied either as an aqueous solution or in gaseous form, but when aqueous solutions are employed the aqueous solution is preferably a saturated or nearly saturated solution.

Any suitable copper compound catalyst can be employed, and many such catalysts are known to the skilled chemist. Preferred catalysts will be copper compounds of the formula:

$Cu_n X_m$

wherein:
X is a halide, $SO_4$, O, $CO_3$ or AcO;
n is 1 or 2; and
m is 0, 1 or 2.

In the process of Scheme III the nitration reaction can be carried out in the presence of concentrated sulphuric acid or acetic acid. Preferably, the concentration of the nitric acid solution employed is 70% or greater. The reaction can conveniently be carried out in a halogenated aliphatic hydrocarbon, in a temperature range between -10 °C and +60 °C.

The above and other characteristics and advantages of the invention will be better understood through the following illustrative and non-limitative description of preferred embodiments thereof.

## Example 1

Preparation of 3-nitrobromobenzene

The preparation is carried out according to J.R. Johnson and C.G. Gauerke, "Organic Synthesis", Coll. Vol. 1, pp. 123 - 124, J. Wiley and Sons, New York, 1956.

Into a 3-necked round-bottomed flask (3 liter), equipped with a mechanical stirrer and a dropping funnel there were introduced:
nitrobenzene 270 g
The flask was heated to 135 - 145 °C and the following reagents:

| | |
|---|---|
| iron (powder) | 26 g |
| bromine | 562 g |

were added in 4 portions within 4 hours with vigorous stirring. The heating continued during an additional hour. The reaction product was poured into water (1.5 liter) to which a saturated solution of $NaHSO_3$ (50 cc) had been added. The mixture was distilled with steam and the first portion of the distillate was collected separately to remove a small amount of unreacted nitrobenzene. Another 12 liters of mixture were steam distilled until all the 3-bromonitrobenzene was obtained. The yellow crystalline solid was filtered and dried to yield 340 g (75% yield) of 3-bromonitrobenzene.

## Example 2

Preparation of 3-nitrodiphenyl ether

The preparation was carried out according to F. Ullmann et al., Justus Liebig's Annalen der Chemie, 350, 83, 1906.

Into a three-necked round-bottomed flask, equipped with azeotropic distillation head, there were introduced:

| | |
|---|---|
| potassium hydroxide (85%) | 8.8 g |
| phenol | 77.1 g |

under nitrogen atmosphere. The mixture was heated to 111 °C and then toluene (50 cc) was added. The water was removed and:
3-bromonitrobenzene 30.1 g

was added, followed by copper catalyst (0.8 g). The reaction mixture was heated to 150 ° C for 3 hours, and to 180 ° C for an additional 30 minutes, until the reaction was completed. The crude mixture was cooled to 80 ° C and washed with 500 cc of 10% NaOH (aq.) and water (1000 cc), to remove the excess phenol. The organic phase was extracted with $CH_2Cl_2$ (3 x 50 cc). The solvent was evaporated in vacuo to obtain 32 g of crude product, which showed the following GC analysis:

| | |
|---|---|
| 3-nitrodiphenyl ether | 90.7% |
| nitrobenzene | 6.5% |
| 3-bromonitrobenzene | 2.7% |

**Example 3**

Preparation of 3-nitro-4'-bromodiphenyl ether

Into a 3-necked round-bottomed flask (500 cc), equipped with a mechanical stirrer and a dropping funnel there were introduced:

| | |
|---|---|
| 3-nitrodiphenyl ether | 27.84 g and |
| $CH_2Cl_2$ | 120 ml |

The solution was stirred at a temperature of 30 - 40 ° C and a solution consisting of:

| | |
|---|---|
| $Br_2$ | 21.75 g |
| $CH_2Cl_2$ | 20 ml |

was added dropwise via a dropping funnel, into the stirred solution in the reactor, during 90 min. The reaction mixture was stirred for 2 additional hours at room temperature. After the completion of the bromination, the excess bromine was removed using $NaHSO_3$ (aq.) until the red color of bromine disappeared. The solution was neutralized using 10% NaOH (aq.) and the phases were separated. The organic phase weighed 36.5 g GC of the crude product showed the following results:

| | |
|---|---|
| 3-nitro-4'-bromodiphenyl ether | 90% |
| 3-nitrodiphenyl ether | 6.3% |
| 3-nitro-o-bromodiphenyl ether | 1.2% |

The crude product was recrystallized from ethanol to obtain the desired product with 99.1% purity (the impurity consisted of 0.9% starting material).

**Example 4**

The bromination of NDPE was performed as in Example 3, but using 1,2-dichloroethane as solvent and at a temperature of 40 - 50 ° C. Substantially the same results, as obtained in Example 3, were obtained.

**Example 5**

Into a 3-necked round-bottomed flask (1 liter), equipped with a mechanical stirrer and a dropping funnel there were introduced:

3-nitrodiphenyl ether 157.7 g

The material was stirred at temperatures of 20 - 50 ° C and 124 g of $Br_2$ were added dropwise via a dropping funnel, into the stirred liquid in the reactor, during 60 min. After completion of the bromination, the excess bromine was removed using 25% $NH_3$ (aq.) until the red color of the bromine disappeared. The phases were separated. The organic phase weighed 206 g GC of the crude product showed the following results:

| | |
|---|---|
| 3-nitro-4'-bromodiphenyl ether | 95.0% |
| 3-nitrodiphenyl ether | 1.0% |
| 3-nitro-2'-bromodiphenyl ether | 2.4% |
| 3-nitro-2',4'-dibromodiphenyl ether | 1.0% |

**Example 6**

Preparation of 3-nitro-4'-aminodiphenyl ether

Into a Parr pressure vessel (1 liter) there were introduced:

| | |
|---|---|
| 3-nitro-4'-bromodiphenyl ether (crude) | 20 g |
| 25% $NH_3$(aq.) | 450 ml |
| $CuSO_4 \bullet 5H_2O$ | 1 g |

The mixture was stirred at 780 - 800 psi for 2.5 hours at 190 °C. After the reaction was completed, the product was extracted with $CH_2Cl_2$. The aqueous phase was titrated to analyze the bromine ions. The titration showed 98% conversion of the starting material. The organic phase was evaporated to obtain 15.4 g black oil which GC analysis showed to contain:

| | |
|---|---|
| 3-nitro-4'-aminodiphenyl ether | 97.05% |
| 3-nitro-2'-aminodiphenyl ether | 0.31% |
| 3-nitro-4'-hydroxydiphenyl ether | 1.3% |
| 3-nitro-4'-bromodiphenyl ether | 1% |

**Example 7**

Preparation of 3,4'-diaminodiphenyl ether

Into a 3-necked round-bottomed flask (150 cc), equipped with a mechanical stirrer there were introduced:

| | |
|---|---|
| 3-nitro-4'-aminodiphenyl ether (97%) | 10.6g |
| $Na_2S \bullet 7\text{-}9\ H_2O$ | 22.5 g |
| $H_2O$ | 31 g |
| Ethanol | 23 g |

The reaction mixture was heated to reflux and after 35 minutes no starting material was observed. The reaction mixture was cooled to room temperature and the product was extracted with ethyl acetate (100 ml). The phases were separated and the solvent was evaporated in vacuo to obtain 15.9 g crude product. The crude product was crystallized from toluene in the presence of active carbon. The end product was distilled to obtain white crystals. GC analysis of the crystals showed the desired product, i.e. 3,4'-diaminodiphenyl ether with 99.9% purity.

**Example 8**

Preparation of 3-amino-4'-bromodiphenyl ether

Into a 3-necked round-bottomed flask (500 ml), equipped with a mechanical stirrer, there were introduced:

| | |
|---|---|
| 3-nitro-4'-bromodiphenyl ether<br>$Na_2S\cdot 7\text{-}9\ H_2O$<br>Ethanol<br>$H_2O$ | 294 g<br>480 g<br>600 ml<br>1200 ml |

The reaction mixture was heated to reflux and after 3.5 hours no starting material was observed. Most of the ethanol and water were distilled out and the crude mixture was cooled to room temperature and extracted with dichloromethane (3 x 300 ml). The solvent was evaporated in vacuo to obtain 256 g crude product. 3-Amino-4'-bromodiphenyl ether was characterized, and its mass spectrogram is shown in Fig. 1. GC analysis showed the following results:

| | |
|---|---|
| 3-amino-4'-bromodiphenyl ether<br>3-nitro-4'-bromodiphenyl ether | 98.3%<br>1.7% |

**Example 9**

Preparation of 3,4'-diaminodiphenyl ether

Into a Parr pressure vessel (1 liter) there were introduced:

| | |
|---|---|
| 3-amino-4'-bromodiphenyl ether<br>25% $NH_3$ (aq.)<br>$CuSO_4\cdot 5H_2O$ | 51.3 g<br>450 ml<br>5.2g |

The mixture was stirred at 1000 psi for 4 hours at 220°C. After the reaction was completed, the product was extracted with $CH_2Cl_2$. The organic phase was evaporated to obtain 32.2 g black oil which GC analysis showed to contain:

| | |
|---|---|
| 3,4'-diaminodiphenyl ether<br>3-aminodiphenyl ether<br>3-amino-4'-bromodiphenyl ether | 96.4%<br>2.0%<br>0.8% |

**Example 10**

Preparation of 3,4'-dinitrodiphenyl ether

Into a 3-necked round-bottomed flask (150 ml) equipped with a mechanical stirrer and a dropping funnel there were introduced:

| | |
|---|---|
| 3-nitrodiphenyl ether<br>Acetic anhydride | 10.75 g<br>40 ml |

The solution was stirred at a temperature of 45 - 50°C and the nitration mixture consisting of:

| | |
|---|---|
| $HNO_3$ (70%)<br>$CH_3COOH$ (100%) | 15 ml<br>15 ml |

was added dropwise via a dropping funnel into the stirred solution in the reactor for 30 minutes. Subsequently the reaction mixture was stirred at the same temperature for one hour and the product was precipitated as a crystalline solid. The precipitate was then filtered, washed with cold water and dried to

obtain 7.6 g yellow crystals. GC analysis showed the following results:

| | |
|---|---|
| 3,4'-dinitrodiphenyl ether | 92% |
| 2,3'-dinitrodiphenyl ether | 4.5% |
| 3-nitrodiphenyl ether | 1.4% |
| non-identified products | 2.1% |

Water addition to remaining filtrate led to the appearance of a precipitate. Its GC analysis showed the following results:

| | |
|---|---|
| 2,3'-dinitrodiphenyl ether | 78.7% |
| 3,4'-dinitrodiphenyl ether | 13.2% |
| 3-nitrodiphenyl ether | 8.1% |

Recrystallization from glacial acetic acid of the overall quantity of precipitate led to 8.8 g of the white crystals. According to GC analysis, the content of 3,4'-dinitrodiphenyl ether is 95.3% and that of 3-nitrodiphenyl ether is 3.7%. The 2,3'-dinitrodiphenyl ether remained in the solvent.

**Example 11**

Into a 3-necked round-bottomed flask (150 ml) equipped with a mechanical stirrer and a dropping funnel, there were introduced:

| | |
|---|---|
| 3-nitrodiphenyl ether | 15 g |
| $CH_2Cl_2$ | 36 ml |

The solution was stirred at a temperature of -10° - 0°C and the nitration mixture consisting of:

| | |
|---|---|
| $HNO_3$ (70%) | 14.4 ml |
| $H_2SO_4$ (98%) | 14.4 ml |

was added dropwise via a dropping funnel into the stirred solution for 30 minutes. Subsequently the reaction mixture was stirred at the same temperature for two hours. Organic substances were extracted by $CH_2Cl_2$, then the solvent was distilled off, and remaining solid (18.1 g) consisting of 3,4'-and 2,3'- isomers was recrystallized from glacial acetic acid to obtain 11.8 g white crystals with the content of 3,4'-dinitrodiphenyl ether 98%.

**Example 12**

Preparation of 3,4'-diaminodiphenyl ether

Into a 3-necked round-bottomed flask (150 ml), equipped with a mechanical stirrer there were introduced:

| | |
|---|---|
| 3,4'-dinitrodiphenyl ether (95.3%) | 8.8 g |
| $Na_2S\cdot 7\text{-}9\ H_2O$ | 31.2 g |
| $H_2O$ | 30 g |
| Ethanol | 24 g |

The reaction mixture was heated to reflux and after 60 minutes, starting material was not observed. The reaction mixture was cooled to room temperature and the product was extracted with ethylacetate (100 ml). The phases were separated and solvent was evaporated in vacuo to obtain 8.1 g crude product. GC analysis showed the 94% content of 3,4'-diaminodiphenyl ether. This crude solid product can be recrystallized.

**Example 13**

Preparation of 3,4'-diaminodiphenyl ether in one step

Into a Parr pressure vessel (1 liter) there were introduced:

| | |
|---|---|
| 3-nitro-4'bromodiphenyl ether | 20.0 g |
| 25% $NH_3$ (aqueous) | 450 ml |
| $CuSO_4 \cdot 5H_2O$ | 1.4 g |

The mixture was stirred at 780 psi for 3 hours at 200 °C. After the reaction was completed, 32.6 g of $Na_2S \cdot 9H2O$ were added to the reactor and the reaction continued at 460 psi (200 °C) for 1 additional hour. GC analysis of the crude product showed:

| | |
|---|---|
| 3,4'-diaminodiphenyl ether | 91.4% |
| 3-aminodiphenyl ether | 7.6% |

## Claims

**1.** A process for the preparation of 3,4'-diaminodiphenyl ether, comprising reacting 3-nitrodiphenyl ether with a compound selected from $Br_2$ and $HNO_3$ to yield a compound of the formula:

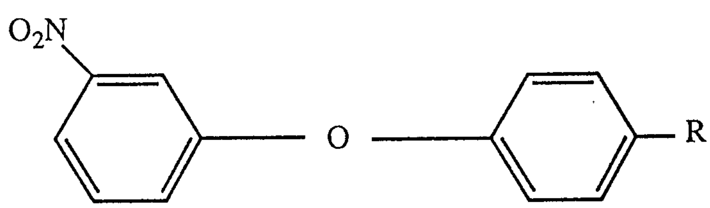

wherein
R is Br or $NO_2$; and then carrying out, in any appropriate order, the steps of:
(a) substituting any Br atom in the 4'-position with an $NH_2$ group;
(b) reducing any $NO_2$ group to an $NH_2$ group.

**2.** A process according to claim 1, comprising brominating 3-nitrodiphenyl ether to yield a compound of formula II:

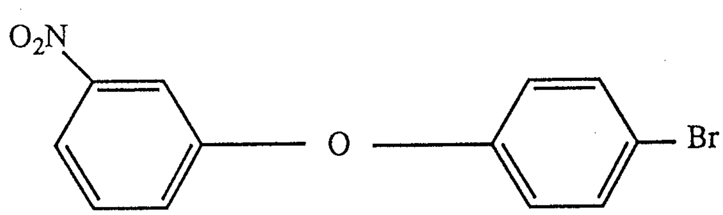

(II)

reacting said compound of formula II with $NH_3$ to yield a compound of formula III:

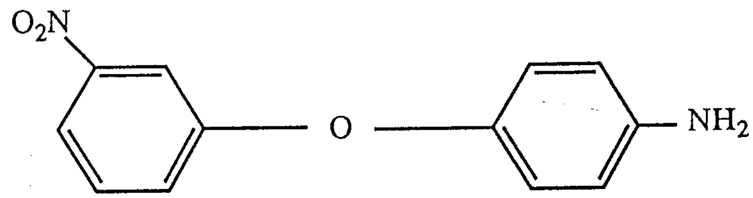

(III)

and reducing the said compound of formula III, to yield 3,4'-diaminodiphenyl ether.

3. A process according to claim 1, comprising brominating 3-nitrodiphenyl ether to yield a compound of formula II:

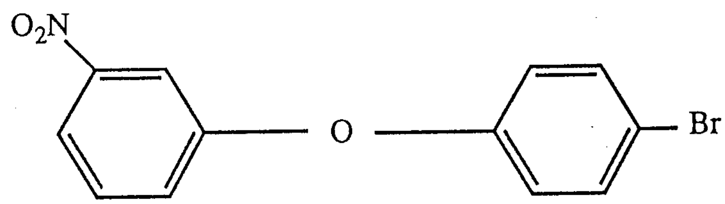

(II)

reducing the said compound of formula II to yield a compound of formula IIIa:

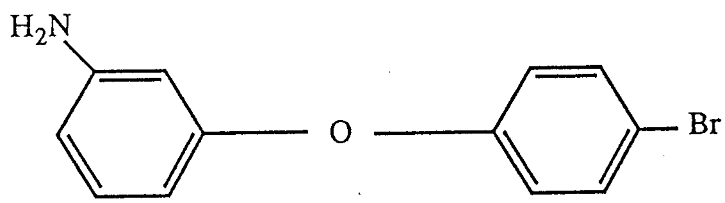

(IIIa)

and reacting the said compound of formula IIIa with $NH_3$, to yield 3,4'-diaminodiphenyl ether.

4. A process according to claim 1, comprising reacting 3-nitrodiphenyl ether with $HNO_3$ to yield a compound of formula IIIb:

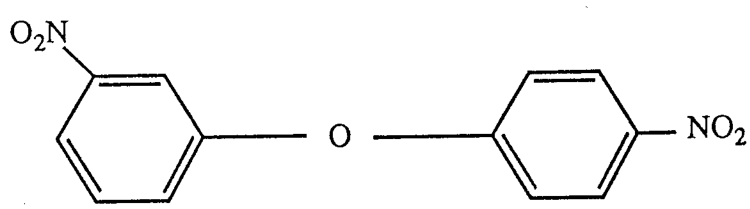

(IIIb)

and reducing the said compound of formula IIIb to yield 3,4'-diaminodiphenyl ether.

5. A process according to claim 2 or 3, wherein the bromination reaction is carried out in a water-immiscible solvent.

6. A process according to claim 5, wherein the solvent comprises a halogenated aliphatic hydrocarbon.

7. A process according to claim 2 or 3, wherein the bromination reaction is carried out in acetonitrile as the solvent.

**8.** A process according to claim 5 or 7, wherein the brominating agent is DBDMH or NBS.

**9.** A process according to claim 2 or 3, wherein the bromination reaction is carried out under neat conditions, substantially in the absence of a solvent.

**10.** A process according to any one of claims 2, 3 and 5 - 9, wherein the bromination reaction is carried out in a temperature range between $^{-}10°$ and $^{+}70°C$.

**11.** A process according to claim 10, wherein the bromination reaction is carried out at a temperature between 20° and 65°C.

**12.** A process according to any one of claims 2, 3 and 5 - 11, wherein the brominating agent is selected from $Br_2$, HBr, alone or together with $H_2O_2$, $NaBrO_3$ or the like oxidizing agents, and organic bromine carriers.

**13.** A process according to claim 12, wherein the organic bromine carriers comprise DBDMH and NBS.

**14.** A process according to any one of the preceding claims, wherein the substitution of the bromine atom with an amino group is carried out by reacting the bromine-containing starting material with ammonia, in an aqueous medium and in the presence of a copper compound catalyst.

**15.** A process according to claim 14, wherein the reaction is carried out at elevated temperatures and under autogenous pressure.

**16.** A process according to claim 14 or 15, wherein ammonia is provided as an aqueous solution or in gaseous form.

**17.** A process according to claim 12, wherein the aqueous solution is a saturated solution.

**18.** A process according to any one of claims 14 to 17, wherein the catalyst is a copper compound catalyst of the formula:

$Cu_n X_m$

wherein
X is a halide, $SO_4$, O, $CO_3$ or AcO;
n is 1 or 2; and
m is 0, 1 or 2.

**19.** A process according to any one of claims 14 to 18, wherein the reaction is carried out at a temperature between 150°C and 300°C, preferably 180° - 250°C.

**20.** A process according to claim 1 or 2, wherein the aminated intermediate is produced and then reduced *in situ.*

**21.** A process according to claim 4, wherein the nitration reaction is carried out with nitric acid in the presence of concentrated sulphuric acid or acetic acid.

**22.** A process according to claim 21, wherein the concentration of the nitric acid solution is 70% or greater.

**23.** A process according to claims 4 and 21, wherein the solvent comprises a halogenated aliphatic hydrocarbon or acetic anhydride.

**24.** A process according to any one of claims 4 and 21-23, wherein the nitration reaction is carried out in a temperature range between -10°C and +60°C.

**25.** The compound 3-amino-4'-bromo-diphenyl ether of the formula:

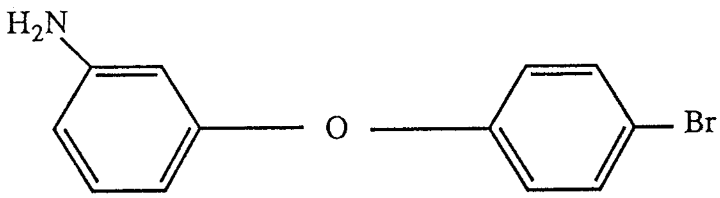

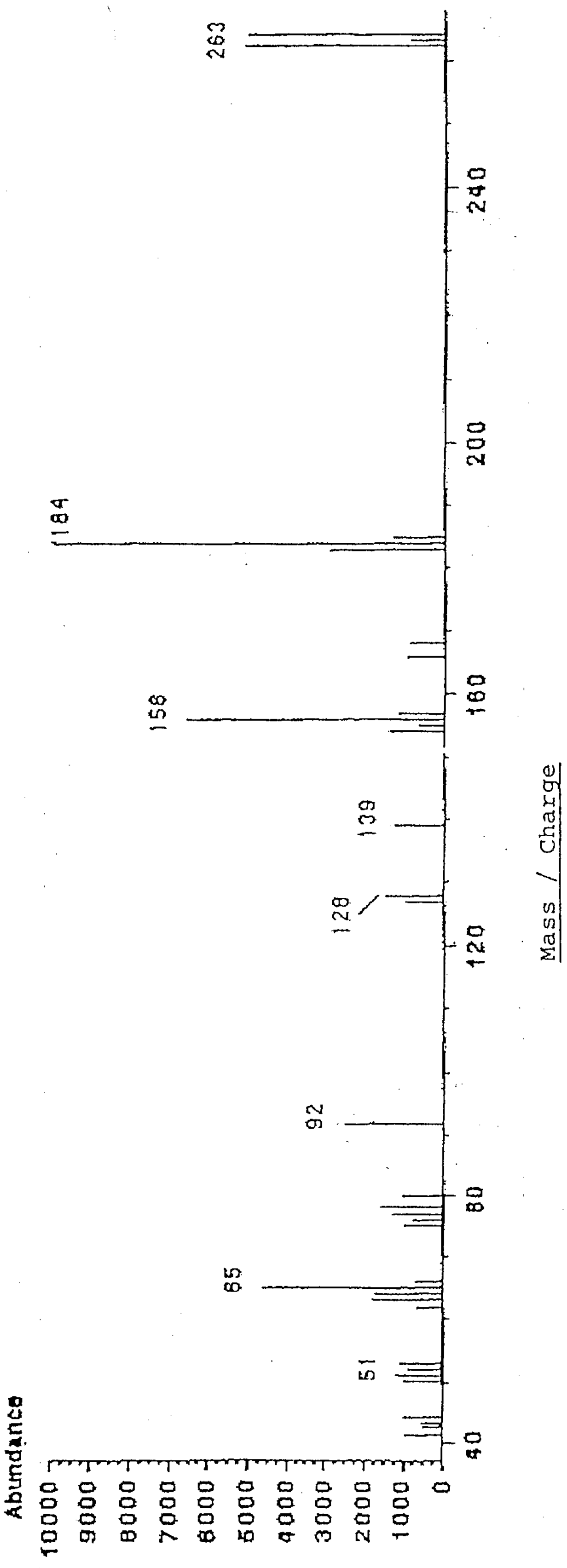
Abundance
10000
9000
8000
7000
6000
5000
4000
3000
2000
1000
0
40
80
120
160
200
240
Mass / Charge
51
65
92
128
139
156
184
263

European Patent Office

# EUROPEAN SEARCH REPORT

Application Number

**EP 91 11 6229**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 140 316 (J.L. TOWLE)<br>* the whole document * * | 1,4,21-24 | C 07 C 205/38<br>C 07 C 201/12<br>C 07 C 201/08<br>C 07 C 217/90<br>C 07 C 213/02 |
| A | US-A-1 890 256 (E.F. GRETHER)<br>* the whole document * * | 1,14-19 | |
| A | PATENT ABSTRACTS OF JAPAN vol. 12, no. 252 (C-512)(3099) 15 July 1988<br>& JP-A-63 039 841 ( TEKUKEMU K.K. ) 20 February 1988<br>* abstract * * | 1,5,6 | |
| A | CHEMICAL ABSTRACTS, vol. 112, 1990, Columbus, Ohio, US; abstract no. 181872, KAWAMURA, M. ET AL.: 'Manufacture of diaminodiphenyl ethers.' page 147 ;column 1 ;<br>* abstract * & JP-A-1 258 651 (MITSUI PETROCHEMICAL INDUSTRIES, LTD.) 16 October 1989 * | 1,2,4 | |
| A | CHEMICAL ABSTRACTS, vol. 109, 1988, Columbus, Ohio, US; abstract no. 191036, SUMITANI, K. ET AL: 'Copper-catalyzed preparation of 3,4'-diaminodiphenyl ether by ammonolysis.' page 11 ;column 1 ;<br>* abstract * & JP-A-6 322 059 (TEIJIN PETROCHEMICAL EP 91116229030INDUSTRIES, LTD.) 29 January 1988 * | 1,14-19 | |

TECHNICAL FIELDS SEARCHED (Int. Cl.5)

C 07 C

The present search report has been drawn up for all claims

| Place of search | Date of completion of search | Examiner |
|---|---|---|
| The Hague | 30 January 92 | BONNEVALLE E.I.H. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document